# EUROPÄISCHE PATENTSCHRIFT

(11) **EP 0 911 020 B1**
(45) Veröffentlichungstag und Bekanntmachung des Hinweises auf die Patenterteilung: **10.03.2004**
(21) Anmeldenummer: 98119094.5
(22) Anmeldetag: 09.10.1998
(51) Int. Cl.: A61K 7/42, A61K 31/275

(54) **Methyl-Styrol derivate als UV-filter und diese enthaltende kosmetische und pharmazeutische Zubereitungen**
Methylstyrene derivatives as UV-filters and cosmetic and pharmaceutical preparations containing them
Dérivés de méthylstyrène comme filtres ultraviolet et compositions cosmétiques et pharmaceutiques les contenant

(30) Priorität: 20.10.1997 DE 19746001; 20.10.1997 DE 19755650
(43) Veröffentlichungstag der Anmeldung: 28.04.1999
(73) Patentinhaber: BASF AKTIENGESELLSCHAFT, 67056 Ludwigshafen (DE)
(72) Erfinder: Habeck, Thorsten, Dr., 67149 Meckenheim (DE); Haremza, Sylke, Dr., 69151 Neckargemünd (DE); Schehlmann, Volker, Dr., 67354 Römerberg (DE); Westenfelder, Horst, 67435 Neustadt (DE); Wünsch, Thomas, Dr., 67346 Speyer (DE)

(56) Entgegenhaltungen:
- EP-A- 0 537 742
- WO-A-91/11989
- DE-A- 1 443 920
- DE-A- 1 914 059
- DE-A- 2 816 819
- DE-B- 1 087 902
- FR-A- 1 368 808
- US-A- 3 278 448
- US-A- 3 522 188
- CHEMICAL ABSTRACTS, vol. 82, no. 25, 23. Juni 1975 Columbus, Ohio, US; abstract no. 170573z, O. L. MNDZHOYAN ET AL: "Derivatives of dicarboxylic acids. XXXIX. alpha-Methyl-alpha-p-alkoxyphenylsuccinimi des" Seite 506; XP002095132 & ARM. KHIM. ZH., Bd. 27, Nr. 12, 1974, Seiten 1056-1061,

## Beschreibung

Die Erfindung betrifft die Verwendung von substituierten α-Methyl-Styrolderivaten als photostabile UV-Filter in kosmetischen und pharmazeutischen Zubereitungen zum Schutz der menschlichen Epidermis oder menschlicher Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PAR-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-Aund UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

In der deutschen Patentschrift Nr. 1 087 902 wird die Verwendung von Kondensationsprodkten aus Oxy- bzw. Alkoxybenzaldehyden und CH-aciden Verbindungen als UV-Filter in technischen Anwendungen, beispielsweise in Kunststoffen beschrieben.

DE 2 816 819 beschreibt 4-Methoxybenzylidencyanessigester als UV-A-Filter in der kosmetischen Anwendung, wobei die in dieser Patentschrift offenbarten Verbindungen nur eine unzureichende Photostabilität aufweisen. Ein weiterer Nachteil ist, daß bei der Synthese der hier beschriebenen UV-Absorber cis/trans-Gemische anfallen, die, wenn sie als solches eingesetzt werden, ein uneinheitliches Gemisch mit unterschiedlichen Absorbtionseigenschaften bilden. Für die Verwendung der reinen Isomeren ist eine aufwendige Aufreinigung erforderlich.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und je nach Substituent in Öl oder Wasser löslich sind.

Diese Aufgabe wurde erfindungsgemäß gelöst durch Verwendung von substituierten α-Methyl-Styrolderivaten der Formel I 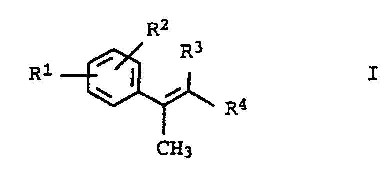 in der die Variablen folgende Bedeutung haben:
- R¹: C₄-C₁₀-Alkoxy, C₁-C₁₂-Alkylamino, C₁-C₁₂-Dialkylamino, wasserlöslich machende Substituenten, ausgewählt aus der Gruppe bestehend aus Carboxylat-, Sulfonat- oder Ammoniumresten;
- R²: Wasserstoff, C₂-C₁₀-Alkyl, C₁-C₁₂-Alkoxy;
- R³: COOR⁵, COR⁵, CONR⁵R⁶, CN;
- R⁴: COOR⁵, COR⁵, CONR⁵R⁶, CN;
wobei die Substituenten R³ und R⁴ jeweils gleich sind;
- R⁵: und
- R⁶: unabhängig voneinander Wasserstoff, C₁-C₁₂-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Alkylreste für R² seien verzweigte oder unverzweigte C₂-C₁₀-Alkylketten, bevorzugt Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl genannt.

Als Alkylreste für R⁵ und R⁶ seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl genannt.

Als Alkenylreste R⁵ und R⁶ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste seien für R⁵ und R⁶ bevorzugt verzweigte oder unverzweigte C₃-C₁₀-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl genannt.

Als Cycloalkenylreste seien für R⁵ und R⁶ bevorzugt verzweigte oder unverzweigte, C₃-C₁₀-Cycloalkenylreste mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecenyl genannt.

Die Cycloalkenyl- und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R¹ kommen solche mit 4 bis 10 C-Atomen, vorzugsweise mit 4 bis 8 C-Atomen in Betracht.

Beispielsweise sind zu nennen:

| | |
|---|---|
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Alkoxyreste für R² kommen solche mit 1 bis 12 C-Atomen, vorzugsweise mit 1 bis 8 C-Atomen in Betracht.

Beispielsweise sind zu nennen:

| | |
|---|---|
| Methoxy- | Ethoxy- |
| Isopropoxy- | -Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Mono- oder Dialkylaminoreste für R¹ und R² kommen solche in Betracht, die Alkylreste mit 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Heteroaryl-Reste sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Hydrophile d.h. die Wasserlöslichkeit der Verbindungen der Formel I ermöglichende Reste für R¹ und R² sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Ammoniumreste, insbesondere Alkylammoniumreste mit beliebigen physiologisch verträglichen Anionen in Betracht.

Bevorzugt sind solche Verbindungen der Formel I, in der
- R¹: C₄-C₆-Alkoxy, insbesondere 3-Methylbutoxy, n-Butoxy und n-Hexoxy,
wasserlöslich machende Substituenten, ausgewählt aus der Gruppe bestehend aus Carboxylat-, Sulfonat- oder Ammoniumresten;
- R²: Wasserstoff, C₂-C₄-Alkyl, Ethyl, n-Propyl, iso-Propyl, C₁-C₈-Alkoxy, insbesondere Methoxy;
- R³: CN
- R⁴: CN;
bedeutet.

Die Substituenten R¹ und R² können jeweils in ortho, meta oder para Position am Aromaten gebunden sein. Besonders bevorzugt sind Verbindungen der Formel I, in denen R¹ in der para- und R² in der meta-Position vorliegen.

Weiterhin weisen Verbindungen der Formel I besondere photostabile Eigenschaften aus, bei denen die Substituenten R¹ bis R⁴ in der in Tabelle 1 genannten Kombination vorliegen:

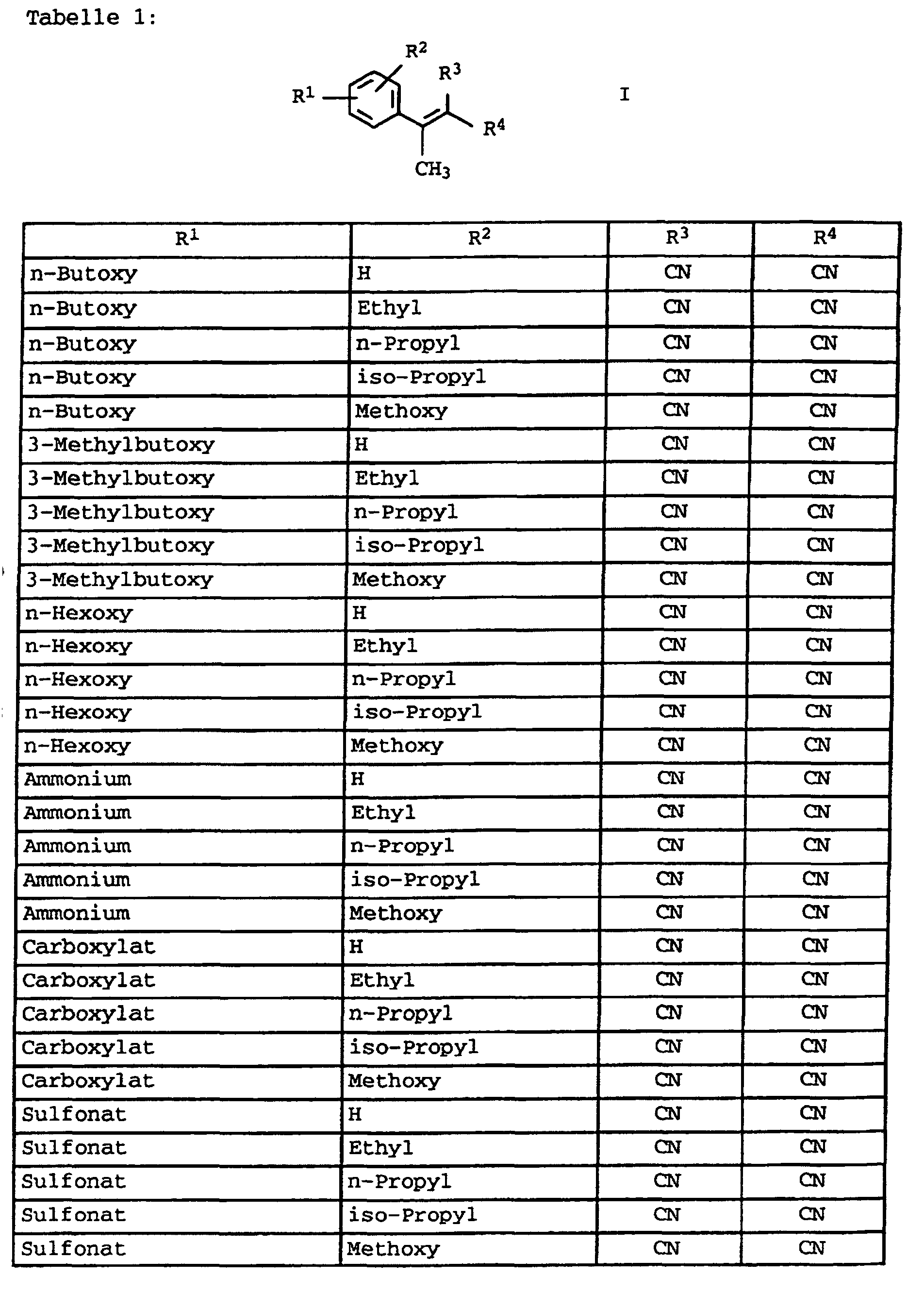

Aus US 3 275 520 sind bereits Verbindungen der allgemeinen Formel II bekannt.

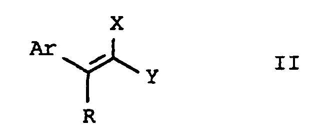

Hinsichtlich des von UV-A-Absorbern geforderten Eigenschaftsprofils, wie u.a. eine gute Öllöslichkeit, eine UV-Stabilität >95% sowie eine hohe spezifische Extinktion konnten nur die erfindungsgemäßen Verbindungen der allgemeinen Formel I diese Bedingungen erfüllen.

Gegenüber den Stand der Technik weisen die erfindungsgemäßen α-Methyl-Styrolderivate der Formel I folgende Vorteile auf:

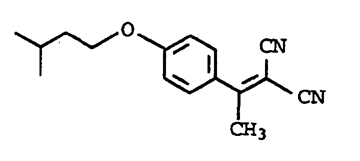

| | |
|---|---|
| Öllöslickeit | > 10% |
| E¹₁ | 723 |
| Photostabilität | > 95% |

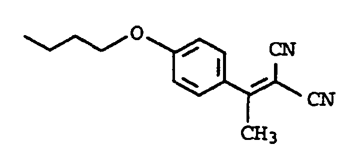

| | |
|---|---|
| Öllöslickeit | > 10% |
| E¹₁ | 678 |
| Photostabilität: | > 95% |

### Vergleichsverbindungen:

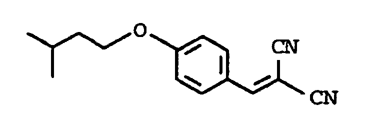

| | |
|---|---|
| Öllöslickeit | > 10% |
| E¹₁ | 950 |
| Photostabilität | 80% |

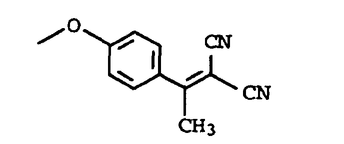

| | |
|---|---|
| Öllöslickeit | < 2% |
| E¹₁ | 800 |
| Photostabilität | > 95% |

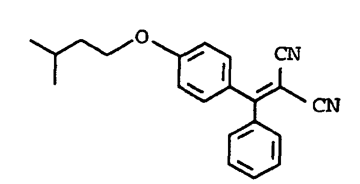

| | |
|---|---|
| Öllöslickeit | < 5% |
| E¹₁ | 550 |
| Photostabilität | > 95% |

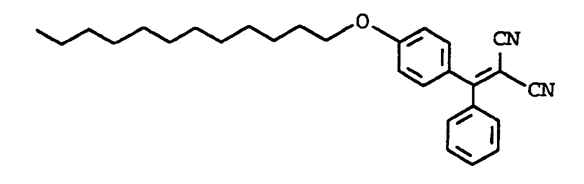

| | |
|---|---|
| Öllöslickeit | > 10% |
| E¹₁ | 400 |
| Photostabilität: | > 95% |

Zusammenfassung [Vorteile gegenüber Stand der Technik (US 3 275 520)]:

| | |
|---|---|
| α-Methyl statt α-Phenyl | bessere Öllöslichkeit, höhere Extinktion |
| α-Methyl statt α-Wasserstoff | bessere Photostabilität |
| (R¹): OC₄H₉ statt OCH₃ | bessere Öllöslichkeit |
| (R¹) : OC₄H₉ statt OC₁₂H₂₅ | höhere Extinktion |

Die erfindungsgemäß zu verwendenden Verbindungen der Formel I können nach der Gleichung 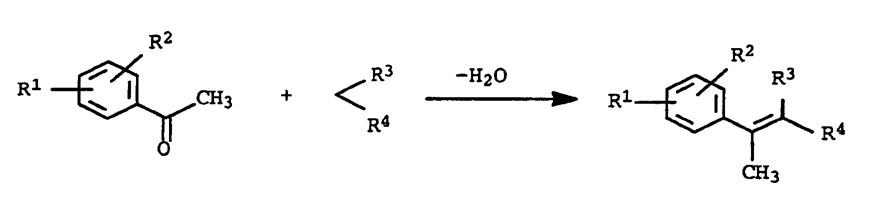 durch Kondensation hergestellt werden, wobei R¹ bis R⁴ die im Anspruch 1 genannte Bedeutung haben.

Beispielsweise ergibt die Umsetzung von 4-(3'-Methylbutoxy)-acetophenon mit Malonsäuredinitril die Verbindung 1 in Tab. 2.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenboman-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo [2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |

Schließlich sind auch mikronisierte Pigmente wie Titandioxid und Zinkoxid zu nennen.

Zum Schutz menschlicher Haare vor UV-Strahlen können die erfindungsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

Gegenstand der Erfindung sind auch die Verbindungen der Formel I zur Verwendung als Medikament sowie pharmazeutische Mittel zur vorbeugenden Behandlung von Entzündungen und Allergien der Haut sowie zur Verhütung bestimmter Hautkrebsarten, welche eine wirksame Menge mindestens einer Verbindung der Formel I als Wirkstoff enthalten.

Das erfindungsgemäße pharmazeutische Mittel kann oral oder topisch verabreicht werden. Für die orale Verabreichung liegt das pharmazeutische Mittel in Form von u.a. Pastillen, Gelatinekapseln, Dragees, als Sirup, Lösung, Emulsion oder Suspension vor. Die topische Anwendung der pharmazeutischen Mittel erfolgt beispielsweise als Salbe, Creme, Gel, Spray, Lösung oder Lotion.

### Beispiele:

### I. Herstellung

### Beispiel 1

### Herstellvorschrift für die Verbindung der Nr. 1 der Tabelle 2

0,1 mol 4-(3'-Methylbutoxy)-acetophenon und 0,1 mol Malonsäuredinitril wurden in 100 ml Toluol gelöst, auf Rückfluß erhitzt und mit 20 ml einer Mischung aus Ammoniumacetat und Eisessig tropfenweise versetzt. Das entstehende Reaktionswasser wurde azeotrop entfernt. Nach dem Abkühlen wurde mit Wasser gewaschen, die organische Phase mit Natriumsulfat getrocknet und im Vakuum eingeengt. Der Rückstand wurde aus Methanol/Wasser kristallisiert.
- Ausbeute:: 7.9 g (33 % d. Th.). Reinheit: > 99 % (GC).

Die Herstellung der Verbindungen 2 bis 4 der Tabelle 2 erfolgt analog Beispiel 1.

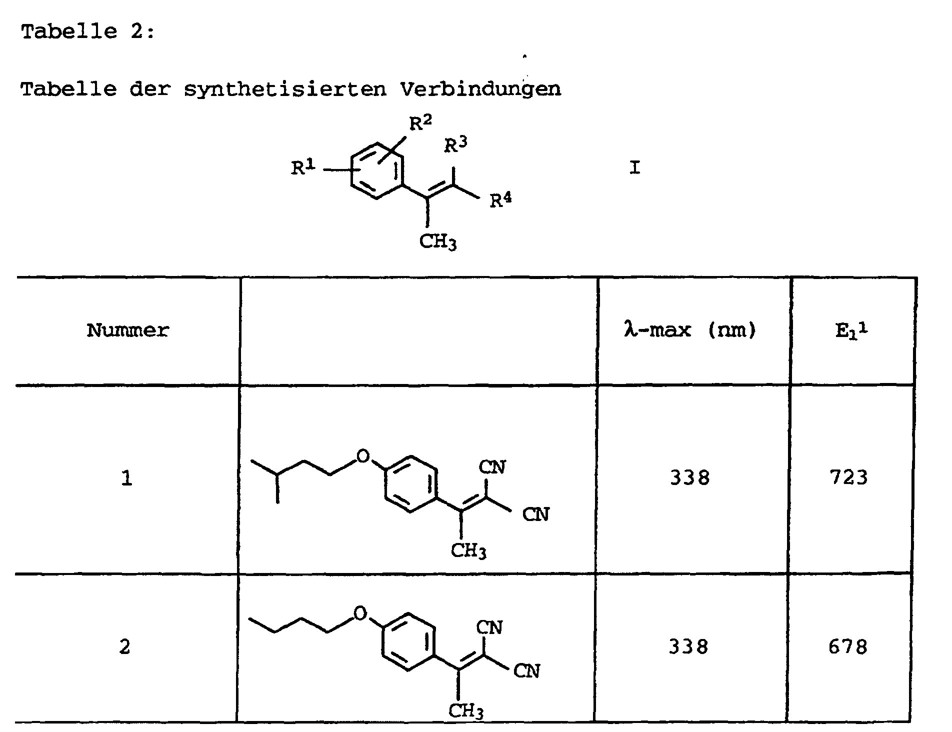

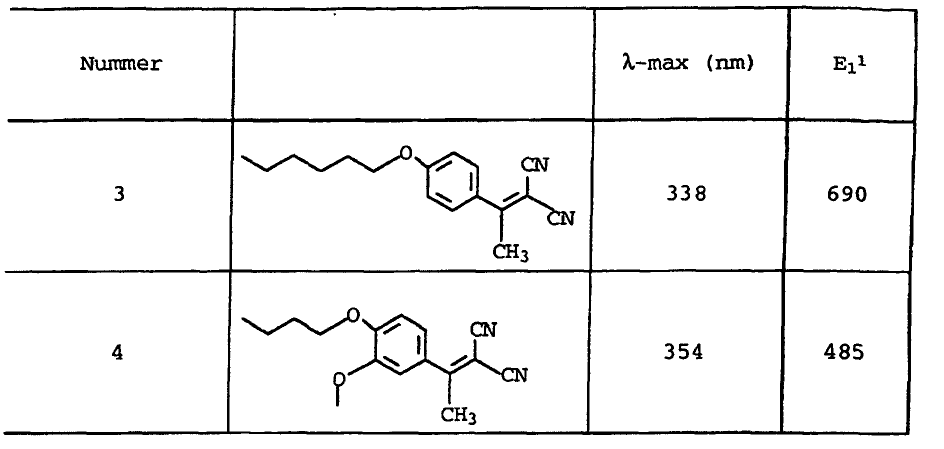

### Beispiel 2

### Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

### Vergleich - Photostabilität:

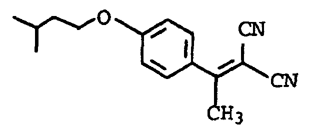

Photostabilität: > 95% 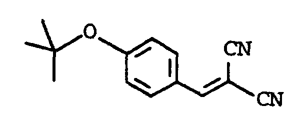 Photostabilität: 72% 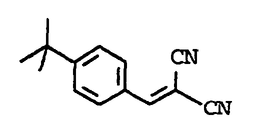 Photostabilität: 81% 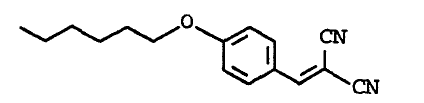 Photostabilität: 78% 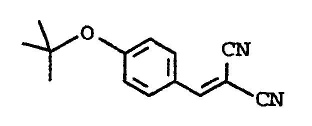 Photostabilität: 81% 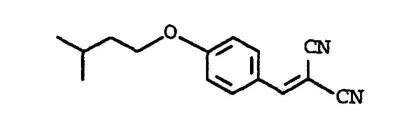 Photostabilität: 80%

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 3

| Zusammensetzung für die Lippenpflege | |
|---|---|
| Massengehalt Gew.-% | |
| ad 100 | Eucerinum anhydricum |
| 10,00 | Glycerin |
| 10,00 | Titanium Dioxid |
| 5,00 | Verbindung Nr. 1 der Tabelle 2 |
| 8,00 | Octyl Methoxycinnamat |
| 5,00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 4

| Zusammensetzung für die Lippenpflege | |
|---|---|
| Massengehalt Gew.-% | |
| ad 100 | Eucerinum anhydricum |
| 10,00 | Glycerin |
| 10,00 | Titanium Dioxid |
| 5,00 | Verbindung Nr. 2 der Tabelle 2 |
| 8,00 | Octyl Methoxycinnamat |
| 5,00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 5

| Zusammensetzung für Sunblocker mit Mikropigmenten | |
|---|---|
| Massengehalt Gew.-% | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 1 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 6

| Zusammensetzung für Sunblocker mit Mikropigmenten | |
|---|---|
| Massengehalt Gew.-% | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 2 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 7

| Fettfreies Gel | |
|---|---|
| Massengehalt Gew.-% | |
| ad 100 | Wasser |
| 8,00 | Octyl Methoxycinnamat |
| 7,00 | Titanium Dioxid |
| 5,00 | Verbindung Nr. 1 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 8

| Fettfreies Gel | |
|---|---|
| Massengehalt Gew.-% | |
| ad 100 | Wasser |
| 8,00 | Octyl Methoxycinnamat |
| 7,00 | Titanium Dioxid |
| 5,00 | Verbindung Nr. 2 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 9

| Sonnencreme (LSF 20) | |
|---|---|
| Massengehalt Gew.-% | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 1 der Tabelle 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 10

| Sonnencreme (LSF 20) | |
|---|---|
| Massengehalt Gew.-% | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 2 der Tabelle 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 11

| Sonnencreme wasserfest | |
|---|---|
| Massengehalt Gew.-% | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 1 der Tabelle 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 12

| Sonnencreme wasserfest | |
|---|---|
| Massengehalt Gew.-% | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 2 der Tabelle 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 13

| Sonnenmilch (LSF 6) | |
|---|---|
| Massengehalt Gew.-% | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 1 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 14

| Sonnenmilch (LSF 6) | |
|---|---|
| Massengehalt Gew.-% | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 2 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von substituierten α-Methyl-Styrolderivaten der Formel I, 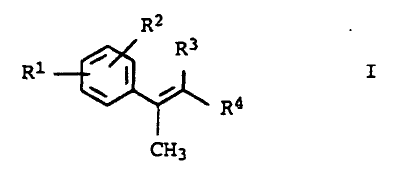 in der die Variablen folgende Bedeutung haben:
R¹ C₄-C₁₀-Alkoxy, C₁-C₁₂-Alkylamino, C₁-C₁₂-Dialkylamino, wasserlöslich machende Substituenten, ausgewählt aus der Gruppe bestehend aus Carboxylat-, Sulfonat- oder Ammoniumresten;
R² Wasserstoff, C₂-C₁₀-Alkyl, C₁-C₁₂-Alkoxy;
R³ COOR⁵, COR⁵, CONR⁵R⁶, CN;
R⁴ COOR⁵, COR⁵, CONR⁵R⁶, CN;
wobei die Substituenten R³ und R⁴ jeweils gleich sind;
R⁵ und
R⁶ unabhängig voneinander Wasserstoff, C₁-C₁₂-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Verwendung von Verbindungen der Formel I gemäß Anspruch 1 als photostabile UV-A-Filter.

3. Verwendung von Verbindungen der Formel I gemäß den Ansprüchen 1 und 2 als UV-Stabilisator in kosmetischen und pharmazeutischen Formulierungen.

4. Verwendung von Verbindungen der Formel I gemäß den Ansprüchen 1 bis 3, wobei die Substituenten folgende Bedeutung haben:
R¹ C₄-C₆-Alkoxy, wasserlöslich machende Substituenten, ausgewählt aus der Gruppe bestehend aus Carboxylat-, Sulfonat- oder Ammoniumresten;
R² Wasserstoff, C₂-C₄-Alkyl, C₁-C₄-Alkoxy;
R³ gleich
R⁴ CN;

5. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit für kosmetische und pharmazeutische Zubereitungen bekannten im UV-Bereich absorbierenden Verbindungen, als photostabile UV-Filter wirksame Mengen von Verbindungen der Formel I 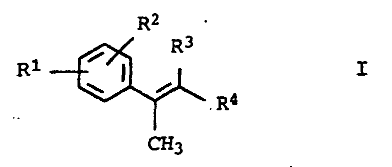 enthalten, in der die Variablen die Bedeutung gemäß Anspruch 1 haben.

6. Lichtschutzmittel enthaltende kosmetische und pharmazeutische Zubereitungen gemäß Anspruch 5, enthaltend als UV-A-Filter Verbindungen der Formel I, in der die Variablen die Bedeutung gemäß Anspruch 4 haben.

7. Verbindungen der Formel I zur Verwendung als Arzneimittel.

8. Pharmazeutische Zubereitung, enthaltend eine wirksame Menge mindestens einer der Verbindung der Formel I nach Anspruch 1.

## Claims

1. The use of substituted α-methylstyrene derivatives of the formula I, 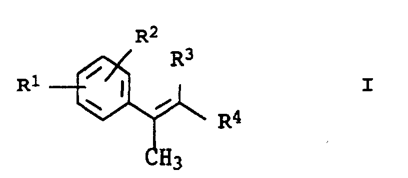 where the variables have the following meanings:
R¹ C₄-C₁₀-alkoxy, C₁-C₁₂-alkylamino, C₁-C₁₂-dialkylamino, substituents which confer solubility in water and which are selected from the group consisting of carboxylate, sulfonate or ammonium residues;
R² hydrogen, C₂-C₁₀-alkyl, C₁-C₁₂-alkoxy;
R³ COOR⁵, COR⁵, CONR⁵R⁶, CN;
R⁴ COOR⁵, COR⁵, CONR⁵R⁶, CN;
where the substituents R³ and R⁴ are identical in each case;
R⁵ and
R⁶ independently of one another hydrogen, C₁-C₁₂-alkyl, C₂-C₁₀-alkenyl, C₃-C₁₀-cycloalkyl, C₃-C₁₀-cycloalkenyl, aryl, hetaryl, unsubstituted or substituted;
as photostable UV filters in cosmetic and pharmaceutical preparations to protect the human skin or human hair from the sun's rays, alone or together with UV-absorbing compounds known per se for cosmetic and pharmaceutical preparations.

2. The use of compounds of the formula I as claimed in claim 1 as photostable UV-A filters.

3. The use of compounds of the formula I as claimed in claims 1 and 2 as UV stabilizers in cosmetic and pharmaceutical formulations.

4. The use of compounds of the formula I as claimed in claims 1 to 3, where the substituents have the following meanings:
R¹ C₄-C₆-alkoxy, substituents which confer solubility in water and which are selected from the group consisting of carboxylate, sulfonate or ammonium residues;
R² hydrogen, C₂-C₄-alkyl, C₁-C₄-alkoxy;
R³ equal to
R⁴ CN.

5. A cosmetic or pharmaceutical preparation comprising sunscreens to protect the human epidermis or human hair from UV light in the range from 280 to 400 nm, which comprises, in a cosmetically or pharmaceutically suitable carrier, alone or together with UV-absorbing compounds known for cosmetic or pharmaceutical preparations, as photostable UV filters, effective amounts of compounds of the formula I 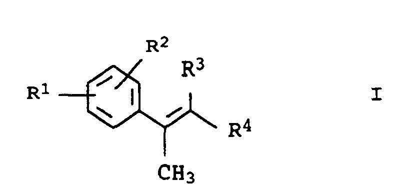 where the variables have the meanings set forth in claim 1.

6. A cosmetic or pharmaceutical preparation comprising sunscreens as claimed in claim 5, which comprises as UV-A filter compounds of the formula I where the variables have the meanings set forth in claim 4.

7. A compound of the formula I for use as drug.

8. A pharmaceutical preparation comprising an effective amount of at least one compound of the formula I as set forth in claim 1.

## Revendications

1. Utilisation de dérivés d'α-méthyl-styrène substitués de formule générale I, 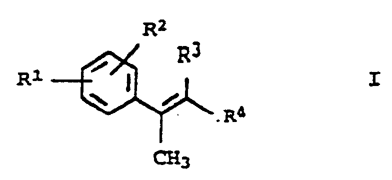 dans laquelle les variables ont la signification suivante:
R¹ alcoxy en C₄-C₁₀, alkyl(C₁-C₁₂)amino, dialkyl(C₁-C₁₂)amino, des substituants de solubilisation dans l'eau choisis dans le groupe consistant en des restes carboxylate, sulfonate ou ammonium;
R² hydrogène, alkyle en C₂-C₁₀, alcoxy en C₁-C₁₂;
R³ COOR⁵, COR⁵, CONR⁵R⁶, CN;
R⁴ COOR⁵, COR⁵, CONR⁵R⁶, CN;
tandis que les substituants R³ et R⁴ sont à chaque fois identiques;
R⁵ et
R⁶ indépendamment l'un de l'autre, hydrogène, alkyle en C₁-C₁₂, alcényle en C₂-C₁₀, cycloalkyle en C₃-C₁₀, cycloalcényle en C₃-C₁₀, aryle, hétéroaryle, éventuellement substitué;
en tant que filtre UV photostable dans des compositions cosmétiques et pharmaceutiques pour la protection de la peau humaine ou de la chevelure humaine contre les rayons solaires, seuls ou en combinaison avec des composés absorbant dans le domaine UV, connus en soi pour des compositions cosmétiques et pharmaceutiques.

2. Utilisation de composés de formule I selon la revendication 1 comme filtre UV-A photostable.

3. Utilisation de composés de formule I selon les revendications 1 et 2 comme stabilisant vis-à-vis des UV dans des compositions cosmétiques et pharmaceutiques.

4. Utilisation de composés de formule I selon les revendications 1 à 3, dans laquelle les substituants ont la signification suivante:
R¹ alcoxy en C₄-C₆, des substituants de solubilisation dans l'eau choisis dans le groupe consistant en des restes carboxylate, sulfonate ou ammonium;
R² hydrogène, alkyle en C₂-C₄, alcoxy en C₁-C₄;
R³ et
R⁴ CN.

5. Compositions cosmétiques et pharmaceutiques contenant des agents de protection contre la lumière pour la protection de l'épiderme humain ou de la chevelure humaine contre la lumière UV dans le domaine de 280 à 400 nm, **caractérisées par le fait qu'**elles contiennent dans un support approprié du point de vue cosmétique et pharmaceutique, seuls ou en combinaison avec des composés absorbant dans le domaine UV, connus pour des compositions cosmétiques et pharmaceutiques, à titre de filtre UV photostable des quantités efficaces de composés de formule I 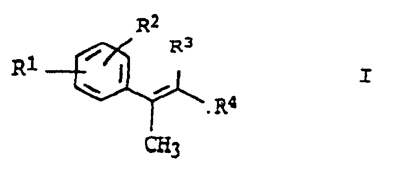 dans laquelle les variables ont la signification selon la revendication 1.

6. Compositions cosmétiques et pharmaceutiques contenant des agents de protection contre la lumière selon la revendication 5, contenant en tant que filtre UV-A des composés de formule I dans laquelle les variables ont la signification selon la revendication 4.

7. Composés de formule I pour utilisation comme médicaments.

8. Composition pharmaceutique contenant une quantité efficace d'au moins l'un des composés de formule I selon la revendication 1.
